# EUROPEAN PATENT APPLICATION

(11) **EP 4 112 081 A1**
(43) Date of publication of application: **04.01.2023**
(21) Application number: 21761588.9
(22) Date of filing: 18.02.2021
(51) Int. Cl.: A61K 47/32, A61K 8/34, A61K 8/37, A61K 8/81, A61K 9/08, A61K 45/00, A61K 47/10, A61K 47/14, A61P 31/02, A61Q 19/10

(54) **DISINFECTING COMPOSITION**

(30) Priority: 26.02.2020 JP 2020030149
(71) Applicant: NOF Corporation, Shibuya-ku Tokyo 150-6019 (JP)
(72) Inventor: HARA, Masao, Kawasaki-shi, Kanagawa 210-0865 (JP); SAKURAI, Shunsuke, Kawasaki-shi, Kanagawa 210-0865 (JP)
(74) Representative: Müller-Boré & Partner Patentanwälte PartG mbB
(86) International application number: PCT/JP2021/006125
(87) International publication number: WO 2021/172162

(57) **Abstract**

The present invention is a disinfecting composition containing 0.001 mass% to 5 mass% of an MPC copolymer containing a constituent unit based on 2-(meth)acryloyloxyethyl phosphorylcholine, 5 mass% to 90 mass% of a lower alcohol, and 0.01 mass% to 10 mass% of an aliphatic acid ester-based nonionic surfactant, wherein the MPC copolymer comprises one or more kind of copolymer selected from the group consisting of an MPC copolymer a having a weight average molecular weight of 10,000 to 400,000 and an MPC copolymer b having a weight average molecular weight of 800,000 to 1,800,000. According to the present invention, a disinfecting composition that is excellent in foam sustainability when it is discharged as a foam and that has moist feeling when it is applied, can be provided.

## Description

### Technical Field

The present invention relates to a disinfecting composition having excellent usage sensation.

### Background Art

In recent years, spreading of infectious diseases caused by pathogenic viruses and bacteria is concerned, and various products relating to prevention of infection are under development. Among these, disinfecting compositions have an advantage that they enable sterilization and disinfection by only applying them to hands and fingers, and thus are widely prevalent.

Disinfecting compositions have been designed so as to impart a broad sterilization spectrum and enhance the sterilization activity, by incorporating a specific sterilization component or adjusting the pH of the compositions to be within a specific region.

For the purpose of improve the usage sensation, such as quick-drying property and non-stickiness while maintaining sterilizing power, there have been devised for the kind of a thickner and adjusting the pH of the composition in a bactericide composition which contains a lower alcohol such as ethanol in a large amount. For example, a gel-like hand sterilizer in which the white turbidity of a composition is prevented by neutralizing a carboxyvinyl polymer with an alkali metal hydroxide and using a polyoxyethylene glycol having a specific molecular weight is suggested (Patent Literature 1).

However, the above-mentioned disinfecting composition did not have a pH in a neutral region, and contained a lower alcohol in a relatively large amount; thus, in the cases here the frequency of use of the disinfecting composition was high as in medical service workers, rough hands might be caused. Therefore, in order to impart an effect to prevent rough hands to a disinfecting composition, further devisal such as incorporation of a moisturizer such as glycerin, propylene glycol or sorbitol (Patent Literature 2), incorporation of a water-soluble polyhydric alcohol and an aliphatic acid triglyceride (Patent Literature 3), etc. have been made.

Furthermore, nowadays, foam-like hand disinfecting compositions are utilized due to their advantages that application sites are easy to notice, and that the compositions are easily spread by application. Foam-like hand disinfecting compositions contain an aliphatic acid ester-based nonionic surfactant as a foaming agent, and are sucked up through a mesh by a pump and discharged as an aggregate of fine bubbles. Lower alcohols are incorporated also in foam-like hand disinfecting compositions for the purposes of application property and quick-drying property; however, since lower alcohols are also defoaming agents, there was a problem that the sustainability of the foam of the discharged hand disinfecting composition was poor, and the foam disappeared before applying it to the hands and fingers and spreading it thereon.

### Citation List

### Patent Literature

PTL 1: JP-A-2011-144146
PTL 2: JP-A-2004-155712
PTL 3: JP-A-2011-219410

### Summary of Invention

### Technical Problem

Therefore, the object of the present invention is to provide a disinfecting composition that has moist feeling when it is applied to the skin and that has foam sustainability when it is discharged, in view of prevention of rough hands, etc.

### Solution to Problem

The present inventors proceeded with intensive studies so as to solve the above-mentioned problem, and consequently found that, by forming a disinfecting composition containing a copolymer containing a constituent unit based on 2-(meth)acryloyloxyethyl phosphorylcholine (MPC) having a specific molecular weight (hereinafter also represented as "MPC copolymer"), a lower alcohol and a aliphatic acid ester-based nonionic surfactant at specific amounts, the foam sustainability of said disinfecting composition can be improved while having moist feeling, and thus the above-mentioned problem can be solved, and completed the present invention.

Specifically, the disinfecting composition of the present invention is as shown in the following [1] to [3].

[1] A disinfecting composition containing 0.001 mass% to 5 mass% of an MPC copolymer containing a constituent unit based on 2-(meth)acryloyloxyethyl phosphorylcholine, 5 mass% to 90 mass% of a lower alcohol, and 0.01 mass% to 10 mass% of an aliphatic acid ester-based nonionic surfactant, wherein the MPC copolymer includes one or more kind of copolymer selected from the group consisting of an MPC copolymer a having a weight average molecular weight of 10,000 to 400,000 and an MPC copolymer b having a weight average molecular weight of 800,000 to 1,800,000.
[2] The disinfecting composition of [1], wherein the lower alcohol is one or more kind selected from the group consisting of ethanol and isopropanol.
[3] The disinfecting composition of [1] or [2], further containing 0.01 mass% to 5 mass% of a sterilizer.

### Advantageous Effects of Invention

According to the present invention, a disinfecting composition that is excellent in foam sustainability when it is discharged as a foam, and that has moist feeling when it is applied, can be provided. Incidentally, moist feeling means a touch as if moisture is retained on the skin after application of a disinfecting composition (a touch that is excellent in moisture-retentive property).

Accordingly, in the case where a sterilizer is incorporated in the disinfecting composition of the present invention, and the disinfecting composition is used on hands and fingers, etc., the disinfecting composition can be applied without application unevenness, and can have both an excellent sterilization effect, and an excellent effect to prevent rough hands, etc.

### Description of Embodiments

### [Disinfecting composition]

The disinfecting composition of the present invention is a disinfecting composition containing 0.001 mass% to 5 mass% of an MPC copolymer containing a constituent unit based on 2-(meth)acryloyloxyethyl phosphorylcholine, 5 mass% to 90 mass% of a lower alcohol, and 0.01 mass% to 10 mass% of an aliphatic acid ester-based nonionic surfactant, wherein the MPC copolymer includes one or more kind of copolymer selected from the group consisting of an MPC copolymer a having a weight average molecular weight of 10,000 to 400,000 and an MPC copolymer b having a weight average molecular weight of 800,000 to 1,800,000.

Hereinafter the details of the present invention will be explained.

### (MPC Copolymer)

The disinfecting composition of the present invention contains an MPC copolymer containing a constituent unit based on 2-(meth)acryloyloxyethyl phosphorylcholine. The MPC copolymer is a copolymer containing a constituent unit based on MPC, and a constituent unit based on a monomer other than MPC. As the constituent unit based on a monomer other than MPC, a constituent unit based on a hydrophobic monomer, which will be mentioned later, is preferable.

### <Constituent unit based on MPC>

The constituent unit based on MPC is represented by the following Formula (1). Incidentally, in the present specification,"(meth)acryloyl" means "acryloyl or methacryloyl".

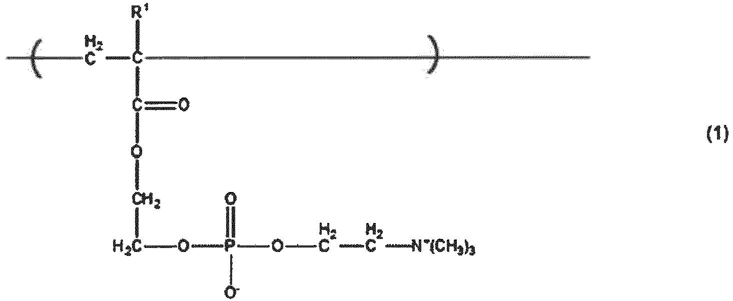

In Formula (1), R¹ represents a hydrogen atom or a methyl group.

### <Constituent unit based on a hydrophobic monomer>

As said constituent unit based on a hydrophobic monomer, constituent units based on a (meth)acrylic acid alkyl ester, (meth)acrylamide derivatives, etc. are exemplified.

The constituent unit based on a (meth)acrylic acid alkyl ester is represented by the following Formula (2). The "(meth)acrylic acid" herein means "acrylic acid or methacrylic acid". 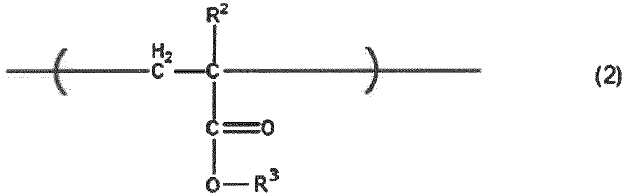 In Formula (2), R² represents a hydrogen atom or a methyl group, and R³ represents an alkyl group having 1 to 24 carbon atoms.

In Formula (2), the alkyl group represented by R³ is a linear or branched alkyl group having 1 to 24 carbon atoms, and part of the hydrogen atoms in the alkyl group has optionally been substituted.

As the linear or branched alkyl group having 1 to 24 carbon atoms, methyl, ethyl, propyl, butyl, pentyl, hexyl, octyl, 2-ethylhexyl, decyl, dodecyl, tetradecyl, hexadecyl, octadecyl, behenyl, etc. are exemplified.

Therefore, the constituent unit based on a (meth)acrylic acid alkyl ester represented by Formula (2) may include constituent units based on methyl (meth)acrylate, ethyl (meth)acrylate, propyl (meth)acrylate, butyl (meth)acrylate, 2-ethylhexyl (meth)acrylate, lauryl (meth)acrylate, tridecyl (meth)acrylate, stearyl (meth)acrylate, behenyl (meth)acrylate, etc.

Furthermore, in the alkyl group represented by R³ in Formula (2), part of the hydrogen atoms in the alkyl group may be substituted. For example, part of the hydrogen atoms in the alkyl group is optionally substituted with a halogen atom, a hydroxy group, an amino group, a quaternary ammonio group, etc.

Therefore, as the constituent unit based on a (meth)acrylic acid alkyl ester, (meth)acrylic acid alkyl esters in which the alkyl group represented by R³ in the above-mentioned Formula (2) has been substituted with a dialkylamino group, a halogen atom, etc. such as diethylaminoethyl (meth)acrylate, trifluoroethyl (meth)acrylate and heptadecafluorodecyl (meth)acrylate, and a constituent unit based on 2-hydroxy-3-(meth)acryloyloxypropyltrimethylammonium chloride in which the alkyl group represented by R³ has been substituted with a hydroxy group and a quaternary ammonio group, are also exemplified as the constituent units based on a hydrophobic monomer.

Among those mentioned above, the constituent unit based on a (meth)acrylic acid alkyl ester is preferably a constituent unit based on butyl (meth)acrylate, lauryl (meth)acrylate, stearyl (meth)acrylate, 2-hydroxy-3-(meth)acryloyloxypropyltrimethylammonium chloride, and is especially preferably a constituent unit based on butyl methacrylate.

The constituent unit based on a (meth)acrylamide derivative is represented by the following Formula (3). The "(meth)acrylamide" herein means "acrylamide or methacrylamide".

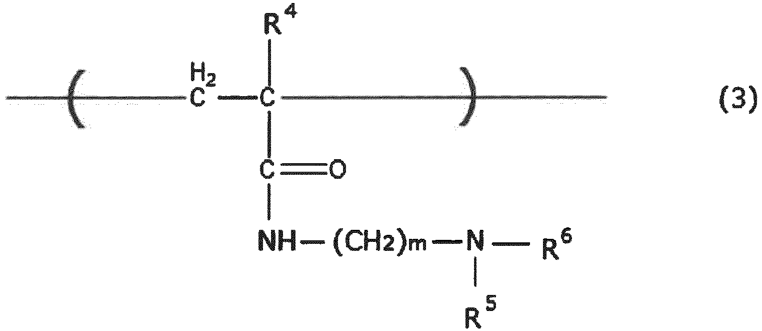

In the formula, R⁴ represents a hydrogen atom or a methyl group, R⁵ and R⁶ each independently represent a methyl group or ethyl group, and m represents an integer of 1 to 3.

For the purpose of the present invention, a copolymer containing a constituent unit based on N,N-dimethylaminoethylacrylamide, or N,N-dimethylaminopropylacrylamide, as the constituent unit based on a (meth)acrylamide derivative, is preferable.

In the present invention, as the MPC copolymer, a copolymer containing a constituent unit based on MPC and one kind or two or more kinds of the above-mentioned constituent units based on a hydrophobic monomer can be used.

For the purpose of the present invention, copolymers containing one kind or two or more kinds selected from the group consisting of constituent units based on a (meth)acrylic acid alkyl ester, and constituent units based on a (meth)acrylamide derivative, as the constituent units based on a hydrophobic monomer, are preferably used.

The ratio of the respective contents of the constituent unit based on MPC and the above-mentioned constituent unit based on a hydrophobic monomer in the MPC copolymer ([constituent unit based on MPC] : [constituent unit based on a hydrophobic monomer]) (n1 : n2) is 9 : 1 to 2 : 1, preferably 8 : 1 to 7 : 3, more preferably 6 : 1 to 3 : 1 by molar ratio.

The polymerization form of the MPC copolymer is not specifically limited, and may be either a random copolymer or a block copolymer, but a random copolymer is preferable.

Furthermore, the MPC copolymer may also be a copolymer containing other constituent unit besides the constituent unit based on MPC and the constituent unit based on a hydrophobic monomer, but a copolymer including only the constituent unit based on MPC and the constituent unit based on a hydrophobic monomer is preferable.

The other constituent unit can be appropriately selected from constituent units that can generally be a constituent unit of the copolymer to the extent that the effect of the present invention is not affected.

Examples of the other constituent unit can include constituent units based on alkenyl esters of (meth)acrylic acid such as allyl (meth)acrylate; cyclic alkyl esters of (meth)acrylic acid such as cyclohexyl (meth)acrylate; (meth)acrylic acid esters having an alkyl group substituted with a hetero ring such as tetrahydrofurfuryl (meth)acrylate; (meth)acrylic acid esters containing an aromatic group such as benzyl (meth)acrylate and phenoxyethyl (meth)acrylate; esters of (meth)acrylic acid and a monoterpene alcohol such as isobornyl (meth)acrylate; glycidyl (meth)acrylate; styrene-based monomers such as styrene, methylstyrene and chloromethylstyrene; vinyl ether-based monomers such as methyl vinyl ether and butyl vinyl ether; and vinyl ester-based monomers such as vinyl acetate and vinyl propionate.

One kind or two or more kinds of these other constituent units may be contained, and the content thereof in the MPC copolymer is 40 mol% or less, preferably 20 mol% or less, more preferably 0 mol% with respect to the total amount of the constituent unit based on MPC and the constituent unit based on a hydrophobic monomer.

The MPC copolymer in the present invention includes one or more kind of copolymer selected from the group consisting of an MPC copolymer a having a weight average molecular weight of 10,000 to 400,000 and an MPC copolymer b having a weight average molecular weight of 800,000 to 1,800,000. That is, the MPC copolymer of the present invention may be the above-mentioned MPC copolymer a, may be the MPC copolymer b, or may also include both the MPC copolymer a and the MPC copolymer b.

In the case where the MPC copolymer does not contain either of the components of the above-mentioned MPC copolymer a and copolymer b, for example, in the case where the MPC copolymer is a copolymer having a weight average molecular weight of greater than 400,000 and lower than 800,000, the foam sustainability decreases when the disinfecting composition is discharged as a foam. Furthermore, in the case where the MPC copolymer is a copolymer having a weight average molecular weight of lower than 10,000, the moisture-retentive property of the disinfecting composition may decrease, and in the case where the MPC copolymer is a copolymer having a weight average molecular weight of greater than 1,800,000, it is possible that a precipitated product is generated and homogeneous application of the disinfecting composition on skin, etc. becomes difficult.

The weight average molecular weight of the MPC copolymer a is preferably 30,000 to 300,000, more preferably 50,000 to 200,000.

The weight average molecular weight of the MPC copolymer b is preferably 900,000 to 1,600,000, more preferably 1,000,000 to 1,400,000.

Incidentally, the weight average molecular weight of the MPC copolymer is measured by gel permeation chromatography (GPC), and is represented by a molecular weight in terms of polyethylene glycol. The GPC is measured under the following conditions.

### <Measurement conditions>

GPC system: High-speed liquid chromatography system CCP & 8020 series (manufactured by Tosoh Corporation)
Column: OHpak SB-802.5HQ (manufactured by Showa Denko K. K.) and OHpak SB-806HQ (manufactured by Showa Denko K. K.) were connected in series Developing solvent: 20 mM sodium phosphate buffer (pH 7.4)
Detector: Differential refractive index detector
Molecular weight standard: EasiVial PEG/PEO (manufactured by Agilent Technologies)
Flow velocity: 0.5 mL/min
Measurement time: 70 min
Column temperature: 45°C
Sample: the obtained copolymer was diluted with a developing solvent so as to have a final concentration of 0.1% by weight.

The content of the MPC copolymer in the disinfecting composition of the present invention is 0.001 mass% to 5 mass%, and from the viewpoint of improvement of the sustainability and moisturizing effect of the foam, the content is preferably 0.005 mass% to 14 mass%, more preferably 0.01 to 3 mass%, further preferably 0.2 to 2 mass%.

When the content of the MPC copolymer is lower than 0.001 mass%, the moisturizing effect may be insufficient in the disinfecting composition, and when the content of the MPC copolymer is greater than 5 mass%, a precipitated product may be generated.

In the case where the MPC copolymer includes the above-mentioned MPC copolymer a and MPC copolymer b, the compounding ratio of the MPC copolymer a and the MPC copolymer b (MPC copolymer a / MPC copolymer b: weight ratio) is preferably 0.1 to 10, more preferably 0.3 to 3, further preferably 0.5 to 2.

The MPC copolymer can be produced by a known production method.

For example, the MPC copolymer can be produced by polymerizing a monomer mixture containing MPC and a hydrophobic monomer such as a (meth)acrylic acid alkyl ester, and containing, as necessary, a monomer corresponding to the above-mentioned other constituent unit, in the presence of a radical polymerization initiator under an inert gas atmosphere such as nitrogen by a known method such as solution polymerization.

The content ratio of the respective monomers during the production can be a ratio corresponding to the content ratio of the respective constituent units in the MPC copolymer.

The MPC copolymer can be produced by the above-mentioned polymerization method and used, or a product commercially available as a solution or dispersion liquid in water or a polyhydric alcohol can also be used.

For the disinfecting composition of the present invention, one kind of the above-mentioned MPC copolymers may be selected and incorporated solely, or two or more kinds can be selected and incorporated in combination.

The form of the MPC copolymer to be incorporated in the disinfecting composition of the present invention may be either a solid such as a powder, a solution, a dispersion liquid.

### (Lower alcohol)

The lower alcohol to be incorporated in the disinfecting composition of the present invention is a monohydric alcohol having 5 or less carbon atoms, and can be used without specific limitation as long as it can dissolve the MPC copolymer and the sterilizer and can be used on skin, and a monohydric alcohol having 3 or less carbon atoms is preferable, and a monohydric alcohol having 2 or 3 carbon atoms is more preferable.

The lower alcohol that is preferably used in the present invention include ethanol, propanol, isopropanol, etc., and ethanol and isopropanol are used more preferably.

In the present invention, one kind of lower alcohol may be used solely, or two or more kinds may be mixed and used.

When the lower alcohol is incorporated in the disinfecting composition of the present invention, the surface tension of the disinfecting composition is lowered, and the composition becomes easy to spread on skin and uneven sites of medical instruments and the evenness during formation of a coating is improved, and thus the disinfecting composition becomes easy to apply evenly onto skin.

The content of the lower alcohol in the disinfecting composition of the present invention is 5 mass% to 90 mass%, preferably 10 mass% to 70 mass%, preferably 15 mass% to 50 mass%, and preferably 20 mass% to 40 mass%.

When the content of the lower alcohol is lower than 5 mass%, there may be cases where unevenness is caused on the formed coating, and thus the disinfecting composition cannot be applied evenly. On the other hand, when the content of the lower alcohol is greater than 90 mass%, there may be cases where the moisturizing effect by the MPC copolymer cannot be obtained.

### (Aliphatic acid ester-based nonionic surfactant)

As the aliphatic acid ester-based nonionic surfactant to be incorporated in the disinfecting composition of the present invention, glycerin aliphatic acid esters, sorbitan aliphatic acid esters, sucrose aliphatic acid esters, polyoxyethylene aliphatic acid esters, polyoxyethylene aliphatic acid sorbitan esters, etc. can be exemplified. Of these, polyoxyethylene aliphatic acid esters are preferable, and among these, it is especially preferable to use polyoxyl 40 stearate.

For the disinfecting composition of the present invention, one kind of the aliphatic acid ester-based nonionic surfactant may be used solely, or two or more kinds may be selected, mixed and used. The content of the aliphatic acid ester-based nonionic surfactant in the disinfecting composition of the present invention can be appropriately preset depending on the kind of the sterilizer, etc., and is generally 0.01 mass% to 10 mass%, preferably 0.01 mass% to 0.5 mass%.

### (Water)

It is preferable that the disinfecting composition of the present invention contains water. In preparing the disinfecting composition of the present invention, it is preferable to use the above-mentioned lower alcohol as a mixed solvent with water since the tight adhesion property of the MPC copolymer against skin can be improved. This is because the transpiration velocities of the lower alcohol and water are different during the drying of the composition, and thus the hydrophobic groups of the MPC copolymer can be oriented effectively on a skin surface.

As the water to be incorporated in the disinfecting composition of the present invention, water that is generally used in cosmetics or medicaments, etc. can be used, and it is preferable to use ion exchanged water, purified water, water for injection, etc.

The content of the water in the disinfecting composition of the present invention is preferably 1 mass% to 89 mass%, more preferably 15 mass% to 85 mass%.

In the case where the content of the water is lower than 1 mass%, or in the case where the content is greater than 89 mass%, it is not preferable since the tight adhesion property of the MPC copolymer against skin may be decreased.

### (Sterilizer)

It is preferable that the disinfecting composition of the present invention contains a sterilizer. The sterilizer is not specifically limited as long as it is an agent that can kill pathogenic microorganisms or suppress the proliferation thereof and can be used for skin disinfection.

Examples of the sterilizer include cationic surfactants such as benzalkonium chloride, benzethonium chloride and cetyl pyridinium chloride; amphoteric surfactants such as alkyldiaminoethylglycine hydrochlorides; biguanide-based compounds such as chlorhexidine gluconate and chlorhexidine hydrochloride; halogen (iodine) compounds such as iodine ion, iodoform and povidone-iodine; silver-based inorganic compounds such as silver ion; and phenolbased compounds such as cresol and isopropyl methyl phenol.

Benzalkonium chloride, benzethonium chloride, chlorhexidine gluconate, etc. are preferably used in view of their sterilization activity and low stimulus property against skin.

For the disinfecting composition of the present invention, one kind of sterilizer may be used solely, or two or more kinds may be selected, mixed and used.

The content of the sterilizer in the disinfecting composition of the present invention may be appropriately preset depending on the kind of the sterilizer, etc. and is generally 0.01 mass% to 5 mass%, more preferably 0.01 mass% to 0.05 mass%.

### (Moisturizer)

Furthermore, the disinfecting composition of the present invention can contain a moisturizer in a range in which the effect of the present invention is not deteriorated.

Examples of the moisturizer include dihydric alcohols such as ethylene glycol, propylene glycol, dipropylene glycol, 1,3-butylene glycol, 1,4-butylene glycol, pentylene glycol, hexylene glycol, isoprene glycol, 2-ethyl-1,3-hexanediol and isopentyldiol; glycerin; glycerin condensates such as diglycerin and polyglycerin; sugar alcohols such as sorbitol, xylitol, maltitol, mannitol and erythritol; and polyol-based compounds, and one kind can be used solely, or two or more kinds can be used as a mixture.

Of these moisturizers, propylene glycol, dipropylene glycol, 1,3-butylene glycol and glycerin are used more preferably since they have good adaption to skin.

The content of the moisturizer in the disinfecting composition of the present invention is selected within a range in which the effect of the present invention is not deteriorated, and is preferably set to 0.01 mass% to 10 mass%.

Additives that are generally used in external preparations such as disinfecting compositions can further be incorporated in the disinfecting composition of the present invention in a range in which the effect of the present invention is not deteriorated.

Examples of such additives include thickeners, propellants, organic acids, antioxidants, stabilizers, antiseptic agents, metal ion sequestrants, flavor materials, pigments and colorants. One kind or two or more kinds of these additives can be selected and incorporated depending on the purpose.

The disinfecting composition of the present invention can be produced by adding a sterilizer, water, a moisturizer and other general additives as necessary to the above-mentioned MPC copolymer, aliphatic acid ester-based nonionic surfactant and lower alcohol, and appropriately mixing these so as to be homogeneous.

The disinfecting composition of the present invention can be provided in a form such as a liquid, or a semi-solid form such as a paste-like form.

In view of convenience, safeness, etc. during use, it is preferable that the disinfecting composition of the present invention is formed into a liquid form and formed into a product such as a foam dischargeable product filled in a container equipped with a foamer pump or an aerosol container.

In the case where the disinfecting composition of the present invention is used for, for example, hand disinfection, the disinfecting composition can be used by taking it in an appropriate amount (about 0.5 mL to 3 mL), applying it to hands by friction, and drying.

The disinfecting composition of the present invention is excellent in foam sustainability when it is discharged, and has moist feeling when it is discharged. Therefore, in the case where a sterilizer is incorporated in the disinfecting composition of the present invention and the disinfecting composition is used on hands and fingers, etc., the composition can be applied evenly without application unevenness, and can have a moisturizing effect and a sterilization effect in combination.

### Examples

Hereinafter the present invention will further be explained in detail with referring to Examples.

### [Examples 1 toll and Comparative Examples 1 to 3]

The disinfecting compositions of Examples 1 to 11 and Comparative Examples 1 to 3 were prepared as follows according to the formulations shown in Table 1.

A powder of a MPC polymer was added to purified water, and stirred to homogeneous. Subsequently, ethanol as a lower alcohol, polyoxyl 40 stearate as an aliphatic acid ester-based nonionic surfactant and benzalkonium chloride as a sterilizer were added, and stirred to homogeneous to prepare 100 g of a disinfecting composition.

Said disinfecting composition was evaluated for the moist feeling and foam sustainability as mentioned below, and the results are shown in Tables 1 and 2.

The respective components used in Examples and Comparative Examples are as shown below.

### <MPC copolymer a>

As MPC copolymer (1), an MPC-butyl methacrylate copolymer (copolymerization composition ratio of MPC and butyl methacrylate [MPC / butyl methacrylate] (molar ratio) = 80 / 20, weight average molecular weight = 87,000) was used.

As MPC copolymer (2), an MPC-butyl methacrylate copolymer (copolymerization composition ratio of MPC and butyl methacrylate [MPC / butyl methacrylate] (molar ratio) = 80/20, weight average molecular weight = 150,000) was used.

### <MPC copolymer b>

As MPC copolymer (4), an MPC-butyl methacrylate copolymer(copolymerization composition ratio of MPC and butyl methacrylate [MPC / butyl methacrylate] (molar ratio) = 80/20, weight average molecular weight = 1,210,000) was used.

### <Other MPC copolymers>

As MPC copolymer (3), an MPC-butyl methacrylate copolymer (copolymerization composition ratio of MPC and butyl methacrylate [MPC / butyl methacrylate] (molar ratio) = 80/20, weight average molecular weight = 600,000) was used.

### <Aliphatic acid ester-based nonionic surfactant>

Polyoxyl 40 stearate was used as an aliphatic acid ester-based nonionic surfactant.

### <Lower alcohol>

Ethanol was used as a lower alcohol.

### <Sterilizer>

Benzalkonium chloride was used as a sterilizer.

Furthermore, as polyoxyl 40 stearate, ethanol and benzalkonium chloride, general commercially available raw materials for use in external preparations were used.

For the disinfecting compositions of the above-mentioned Examples and Comparative Examples, the moist feeling and foam sustainability were evaluated as follows.

### (1) Evaluation of moist feeling

The moist feeling when the disinfecting compositions of Examples and Comparative Examples were applied on skin were evaluated by sensing evaluation by 20 male and female panelists of 25 to 55 years old.

That is, under an environment at a room temperature of 25°C and a relative humidity of 40%, each panelist was made to take 2 mL of each disinfecting composition, apply it onto their hands and fingers by friction ten times, touch the tips of the fingers by a finger, and evaluate the moist feeling according to the following evaluation criteria. The total of the evaluation points by the 20 panelists was calculated, and the moist feeling was judged by the following judgment criteria.

### <Evaluation criteria>

Feel moist: 2 points
Feel somewhat moist: 1 point
Feel no moist: 0 point

### <Judgment criteria>

31 points to 40 points: A; a disinfecting composition having moist feeling
21 points to 30 points: B; a disinfecting composition having somewhat moist feeling
20 points or less: C; a disinfecting composition with no moist feeling

### (2) Evaluation of foam sustainability

Each of the disinfecting compositions of Examples and Comparative Examples was put into a container, and a pump foamer (M1 Pump Foamer 255/200 mesh, manufactured by Daiwa Can Company) was attached. The composition was discharged under an environment at a room temperature of 25°C and a relative humidity of 40% from a distance of 5 cm above a Kim Towel (manufactured by Nippon Paper Crecia Co., Ltd.) so that the foam had a surface area of 4.5 ± 0.5 cm², and the size of the foam at 90 seconds after the discharge was measured. This measurement was repeated three times, and an average value of the three measured values was calculated. The sustainability of the foam was judged according to the following judgment criteria.

### <Judgment criteria>

1.0 cm² or more: A; a disinfecting composition excellent in foam sustainability
0.8 cm² or more, lower than 1.0 cm²: B; a disinfecting composition somewhat excellent in foam sustainability
Lower than 0.8 cm²: C; a disinfecting composition for which foam sustainability cannot be said to be sufficient

### [Table 1]

**Table 1**

| Components/Evaluation items | | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|
| Disinfecting compositions (mass%) | MPC copolymer (1) | 0.05 | 0.5 | 0.5 | - | - | - | - | - | - | 0.125 | - |
| | MPC copolymer (2) | - | - | - | 0.05 | 0.5 | 0.5 | | | - | - | 0.125 |
| | MPC copolymer (3) | | | | | | | | | | | |
| | MPC copolymer (4) | - | - | - | - | - | | 0.05 | 0.5 | 0.5 | 0.125 | 0.125 |
| | Ethanol | 20 | 20 | 30 | 20 | 20 | 30 | 20 | 20 | 30 | 30 | 30 |
| | Polyoxyl 40 stearate | 0.20 | 0.20 | 0.20 | 0.20 | 0.20 | 0.20 | 0.20 | 0.20 | 0.20 | 0.20 | 0.20 |
| | Benzalkonium chloride | 0.05 | 0.05 | 0.05 | 0.05 | 0.05 | 0.05 | 0.05 | 0.05 | 0.05 | 0.05 | 0.05 |
| | Purified water | Remainder | Remainder | Remainder | Remainder | Remainder | Remainder | Remainder | Remainder | Remainder | Remainder | Remainder |
| | Total | 100 | 100 | 100 | 100 | 100 | 100 | 100 | 100 | 100 | 100 | 100 |
| Evaluation results | Moisture feeling | B | A | A | B | A | A | B | A | A | A | A |
| | Foam sustainability | B | A | A | B | A | A | B | A | A | A | A |

| | | | | | | | | | | | | |
|---|---|---|---|---|---|---|---|---|---|---|---|---|
| * In the table, '-' means that the relevant component is not incorporated. | | | | | | | | | | | | |

### [Table 2]

**Table 2**

| Components/Evaluation items | | Comparative Example 1 | Comparative Example 2 | Comparative Example 3 |
|---|---|---|---|---|
| Disinfecting compositions (mass%) | MPC copolymer (1) | - | - | - |
| | MPC copolymer (2) | - | - | - |
| | MPC copolymer (3) | - | 0.05 | 0.5 |
| | MPC copolymer (4) | - | - | - |
| | Ethanol | 20 | 20 | 20 |
| | Polyoxyl 40 stearate | 0.20 | 0.20 | 0.20 |
| | Benzalkonium chloride | 0.05 | 0.05 | 0.05 |
| | Purified water | Remainder | Remainder | Remainder |
| | Total | 100 | 100 | 100 |
| Evaluation results | Moisture feeling | C | B | A |
| | Foam sustainability | C | C | C |

| | | | | |
|---|---|---|---|---|
| * In the table, '-' means that the relevant component is not incorporated. | | | | |

As represented in Table 1, the respective disinfecting compositions of Examples 1 to 11 of the present invention were evaluated to have moist feeling and to be excellent in foam sustainability. It was recognized that the disinfecting compositions of Examples 2, 3, 5, 6, 8 and 9, which contained 0.5 mass% of the MPC copolymer, were especially excellent in moist feeling and foam sustainability.

To this, the disinfecting composition of Comparative Example 1, which did not contain an MPC copolymer, was evaluated to be a disinfecting agent without moist feeling, and to be a disinfecting agent lacking foam sustainability. The disinfecting compositions of Comparative Examples 2 and 3, which contained an MPC copolymer, but the average molecular weight of the MPC copolymer was out of the specific molecular weight, were evaluated to be disinfecting agent having moist feeling but lacking foam sustainability. The reason is considered that the MPC copolymer outside the specific molecular weight had a relatively low surface tension, and thus the disinfecting agent was not able to surround fine bubbles when it was discharged as a foam and thus was poor in foam sustainability.

From the above-mentioned evaluation results for the respective disinfecting compositions of Examples and Comparative Examples, it was clarified that the disinfecting compositions of the present invention were excellent in both foam sustainability and moist feeling as compared to the disinfecting compositions of Comparative Examples, which did not contain an MPC copolymer.

### Industrial Applicability

As stated above in detail, the present invention can provide a disinfecting composition that has moist feeling, and is excellent in foam sustainability when it is discharged as a foam.

When the disinfecting composition of the present invention is used on hands and fingers, etc., the disinfecting composition is easy to apply when it is discharged, and thus the composition is excellent in usage sensation and can prevent rough hands, etc.

## Claims

1. A disinfecting composition containing 0.001 mass% to 5 mass% of an MPC copolymer containing a constituent unit based on 2-(meth)acryloyloxyethyl phosphorylcholine, 5 mass% to 90 mass% of a lower alcohol, and 0.01 mass% to 10 mass% of an aliphatic acid ester-based nonionic surfactant, wherein the MPC copolymer comprises one or more kind of copolymer selected from the group consisting of an MPC copolymer a having a weight average molecular weight of 10,000 to 400,000 and an MPC copolymer b having a weight average molecular weight of 800,000 to 1,800,000.

2. The disinfecting composition according to claim 1, wherein the lower alcohol is one or more kind selected from the group consisting of ethanol and isopropanol.

3. The disinfecting composition according to claim 1 or 2, further containing 0.01 mass% to 5 mass% of a sterilizer.
